# EUROPEAN PATENT SPECIFICATION

(11) **EP 1 459 740 B1**
(45) Date of publication and mention of the grant of the patent: **06.06.2007**
(21) Application number: 04251530.4
(22) Date of filing: 17.03.2004
(51) Int. Cl.: A61K 9/28

(54) **Compositions containing sucralose**
Zubereitungen enthaltend Sucralose
Composition contenant sucralose

(30) Priority: 18.03.2003 US 391396
(43) Date of publication of application: 22.09.2004
(73) Proprietor: McNEIL-PPC, Inc., Skillman, New Jersey 08558 (US)
(72) Inventor: Szymczak, Christopher E, Marlton, New Jersey 08503 (US)
(74) Representative: Mercer, Christopher Paul

(56) References cited:
- EP-A- 1 297 827
- WO-A-92/17161
- GB-A- 2 249 465
- US-A- 3 992 519
- US-A1- 2003 118 654
- US-B1- 6 497 859

## Description

### FIELD OF THE INVENTION

This invention relates to a composition for coating substrates, such as tablets and capsules, and methods for producing such coated tablets and capsules.

### BACKGROUND OF THE INVENTION

A major concern in designing pharmaceutical dosage forms is making convenient, palatable medications that facilitate patient compliance with the recommended dosing regimen. One of the most popular pharmaceutical dosage forms includes tablets that may be swallowed. It is common practice to coat such dosage forms with substances, such as film-forming polymers, fats, sugars, or gelatin, in order to facilitate swallowing ease, to hide an objectionable taste of the tablet, and/or to provide a perceptible pleasing taste to the tablet.

Designing a dosage form with superior taste, mouth feel, or other organoleptic characteristics, such as one that provides a sensory "cue" to the consumer that the medicine may be starting to work, are all known methods of obtaining a consumer-preferred product. Recently in the confectionary marketplace, mints, gums, and breath-freshening strips, which provide a cooling sensation in the mouth or throat, have also become especially popular with consumers.

In the pharmaceutical marketplace, cooling agents have also been used in dosage forms not only to satisfy the consumer's preference for a pleasant tasting form, but also to enhance the physiological and/or perceived benefits, e.g., speed of relief, duration of relief, and improved aesthetics of the medicine. For example, it is known to include volatile mint-like compounds, such as menthol or peppermint oil, in coatings for swallowable pharmaceutical tablets in order to provide the user with a cooling sensation. See, e.g., U.S. Patent No. 5,098,715 and U.S. Patent No. 5,827,852. However, due to the taste and smell associated with the use of such volatile compounds, the risk of their misuse is significant. Another limitation associated with the use of volatile mint-like compounds is the dietary restrictions regarding mint usage in certain patient populations, e.g. those with gastro-esophageal reflux disease ("GERD"). Yet another limitation regarding the use of such volatile compounds is a perceived social stigma associated with the smell of mentholated medicine in public. Furthermore, dosage forms having a "minty" or menthol-like smell or odor may be confused with candies and mints or cough-drops. In the case of pets that rely on the sense of smell, or visually handicapped, this could also cause accidental ingestion of a medication or confusion with other items normally ingested.

Cooling agents have been also been employed into chewable dosage form in order to create a prolonged cooling sensation in the throat. See PCT Publication No. 97/24036 or PCT Publication No. 92/17161. However, such chewable dosage forms are designed to remain in the mouth for some period of time and may not disintegrate or dissolve completely upon chewing. Not only may this retard dissolution of the active agent, but it also may delay onset of the active.

Coolants or cooling agents have also been employed with sweeteners in liquid cough-treatment compositions. See PCT Publication No. WO 02/45714. However, the portability of liquids limits their use, and some high-intensity sweeteners, such as aspartame, are subject to degradation when heated.

A need therefore remains for an economic, easy-to-swallow dosage form that provides a pleasant cooling sensation substantially absent of any olfactory stimulation and is substantially free of a volatile compound.

### SUMMARY OF THE INVENTION

According to the present invention there is provided a pharmaceutical dosage form as defined in the appendant claims.

A preferred solid dosage form comprises a coating composition comprising
a) a hydroxypropylmethylcellulose coating agent;
b) a sucralose heat-stable, high-intensity sweetening agent; and
c) a menthyl ester non-volatile cooling agent.

We have found that in the dosage form of the present invention, its coating composition masks any unpleasant taste that may be associated with the dosage form and / or the coating agent itself. The coating composition further provides the user with a mild, pleasant cooling sensation in the mouth and throat during ingestion without any substantial aroma or olfactory stimulation.

### DETAILED DESCRIPTION OF THE INVENTION

As used herein, the term "dosage form" applies to any solid, semi-solid, or liquid composition designed to contain a specific pre-determined amount or "dose" of a certain ingredient, for example an active ingredient as defined below. Dosage forms may include, but are not limited to: a) pharmaceutical drug delivery systems, including those for oral administration, buccal administration, rectal administration, topical or mucosal delivery, implants for subcutaneous delivery or other implanted drug delivery systems; or b) compositions for delivering minerals, vitamins and other nutraceuticals, oral care agents, flavorants, and the like. The dosage forms of the present invention are typically considered to be solid; however, they may contain liquid or semi-solid components. Suitable "solid dosage forms" of the present invention include, but are not limited to, tablets such as caplets, capsules, sachets, and the like. One suitable solid dosage form is an elongated tablet commonly referred to as a "caplet". In one embodiment, the solid dosage form is an orally administered system for delivering a pharmaceutical active ingredient to the GI tract.

Dosage forms typically contain a substrate or core. As used herein, the terms "substrate" or "core" may be used interchangeably and refer to a surface or underlying support, upon which another substance at least partially resides or acts. Typically, the core is in the form of a solid such as, for example, a compressed or molded tablet, that is prepared via compression or molding. Alternatively, the core may be in the form of a semi-solid or a liquid in the finished dosage form.

"Hardness," as used herein, describes the diametral breaking strength of either the core or the coated solid dosage form as measured by conventional pharmaceutical hardness testing equipment, such as a Schleuniger Hardness Tester. In order to compare values across different size tablets, the breaking strength must be normalized for the area of the break. This normalized value, expressed in kp/cm², is sometimes referred in the art as "tablet tensile strength." A general discussion of tablet hardness testing is found in Leiberman et al., Pharmaceutical Dosage Forms - Tablets, Volume 2, 2nd ed., Marcel Dekker Inc., 1990, pp. 213 - 217, 327 - 329.

"Coating composition," as used herein, refers to a dry composition in the form of a coating on a dosage form or on a plurality of particles contained in a dosage form.

"Coating solution," as used herein, refers to a fluid coating material in the form of a dispersion, suspension, or emulsion that is suitable for application to the surface of a substrate via, for example, spraying, dipping, or molding. Typically, the coating solution includes a solvent or liquid carrier, which is removed during processing by, for example, drying, to form the final dried coating composition.

In one embodiment, a particularly useful dosage form of the present invention is a coated solid pharmaceutical dosage form such as, for example, a swallowable tablet or caplet. In another embodiment, a useful dosage form of the present invention has the coating on a plurality of particles that contain pharmaceutical active ingredient, and are incorporated into a pharmaceutical dosage form such as, for example, a chewable tablet.

In one embodiment, the dosage form of the present invention comprises a coating composition that may be employed as a taste masking coating for active ingredient particles. Examples of taste masking coatings and methods for applying a taste masking coating onto particles are described in, for example, U.S. Patent No. 4,851,226, U.S. Patent No. 5,075,114, and U.S. Patent No. 5,489,436. In one embodiment, the particles coated with a taste masking composition may be employed as part of a solid dosage form such as, for example, a chewable tablet. In another embodiment, the coated particles may be employed in a multiparticulate solid dosage form such as, for example, sachets, sprinkles and the like.

In embodiments in which the dosage form of the invention comprises a coating composition that is incorporated as a coating on a swallowable solid dosage form, such as a swallowable tablet, the coated dosage form typically has a weight from 50 mg to 2000 mg, e.g. from 100 mg to 1000 mg, with a 0.5 percent to a 4 percent increase in weight relative to an uncoated dosage form.

In embodiments in which the dosage form of the invention comprises a coating composition that is applied onto a particle, each individually-coated particle typically has an average diameter from 10 microns to 2000 microns, for example from 50 microns to 1000 microns or from 100 microns to 800 microns, whereby the thickness of the coating composition may range from about 20 microns to 800 microns, i.e., for e.g., from 50 microns to 125 microns. "Water soluble" as used herein in connection with non-polymeric materials, shall mean from sparingly soluble to very soluble, i.e., not more than 100 parts water required to dissolve 1 part of the non-polymeric, water soluble solute. See Remington, "The Science and Practice of Pharmacy," pages 208 - 209 (2000). "Water soluble" as used herein in connection with polymeric materials, shall mean that the polymer swells in water and can be dispersed at the molecular level to form a homogeneous dispersion.

"Cooling agents," as used herein, include solid or liquid substances that inhibit heat receptors or stimulate cooling receptors located on the free-nerve endings of the CN V trigeminal nerve. In one embodiment, the cooling agents provide a sensory cooling effect, either immediate or delayed, to the user without significant interaction with one or more of the taste sensors such as bitter, sour, sweet, umami, or salty.

"Non-volatile cooling agents," as used herein, shall represent a subgroup of cooling agents comprised of one or more individual chemical compounds that are substantially free from odor and odorless vapor such that they a) do not lose more than about 1% by weight when placed in an open container at 50°C for at least one hour; and usually b) have an average molecular weight of greater than 300 atomic molecular units (amu) or more as described by the "The Royal Society of Chemistry" website, London UK (www.chemsoc.org/ exemplarchem/entries/2001/caphane/flavour.html , 2002). "Average molecular weight," as used herein, shall mean a mathematical weighted average of all of the individual components weighted according to the weight fraction or percent concentration in solution as defined in Martin, Physical Pharmacy, 561 (4^{th} Ed. 1993)(also referred to as "weight-average molecular weight" ).

"Cooling sugars," as described herein, shall include all sugar alcohols that have negative heats of solution ( enthalpy, ΔH < 0 J/mol ) and are known to impart some cooling sensation when placed upon a tongue of a user.

"Cooling adjuvants," as described herein, shall refer to all compounds that have a negative heats of solution ( i.e., an enthalpy, ΔH of less than 0 J/mol). Examples of suitable cooling adjuvants include, but are not limited to cooling sugars.

The first embodiment of this invention is directed to a dosage form that comprises a coating composition including, based upon the total weight of the coating composition, a) from 3 percent to 99.93 percent, e.g. from 65 percent to 85 percent of a coating agent selected from the group consisting of film forming polymers, fats having a melting point less than 80 °C, waxes having a melting point less than 80 °C, and mixtures thereof ; b) from 0.01 percent to 89 percent, e.g. from 0.01 percent to 25 percent of a heat stable, high-intensity sweetening agent; c) from 0.01 percent to 92 percent, e.g. from 0.01 percent to 37 percent of a non-volatile cooling agent; and d) from 0 percent to 37 percent, e.g. from 0 percent to 25 percent of a cooling adjuvant.

Yet another embodiment of this invention is directed to a dosage form that comprises a coating composition including, based upon the total weight of the coating composition, a) from 38 percent to 99.97 percent, e.g. from 62 percent to 99.97 percent of a crystallizable carbohydrate coating agent; b) from 0.01 percent to 29 percent, e.g. from 0.01 percent to 17 percent of a heat stable, high-intensity sweetening agent; and c) from 0.01 percent to 38 percent, e.g. from 0.01 percent to 25 percent of a non-volatile cooling agent.

In one embodiment, the coating composition is substantially free of volatile cooling agents such as mint and menthol. "Substantially free of volatile cooling agents," as used herein, shall mean inclusion of less 0.1 percent, e.g., less than 0.01 percent, of volatile cooling agents as based upon the total weight of the coating composition.

Suitable coating agents include, but are not limited to crystallizable carbohydrates such as sucrose, dextrose, fructose, maltodextrin, polydextrose, and cooling sugars such as sorbitol, erythritol, lactitol, maltitol, mannitol, xylitol , and mixtures thereof. Other suitable coating agents include, but are not limited to film forming polymers; waxes having a melting point less than 80 °C such as polyethylene glycol, bees wax, shellac wax, camuba wax, candela wax, and microcrystalline wax; fats having a melting point less than 80 °C; and mixtures thereof.

Any film forming polymer known in the art is suitable for use in the coating composition of the present invention. Examples of suitable film forming polymers include, but are not limited to, polyvinylalcohol (PVA), hydroxypropyl starch, hydroxyethyl starch, pullulan, methylethyl starch, carboxymethyl starch, methylcellulose, hydroxypropylcellulose (IIPC), hydroxyethylmethylcellulose (HEMC), hydroxypropylmethylcellulose (HPMC), hydroxybutylmethylcellulose (HBMC), cellulose acetate (CA), cellulose acetate phthalate (CAP), carboxymethylcellulose (CMC), hydroxyethylethylcellulose (HEEC), hydroxyethylhydroxypropylmethyl cellulose (HEMPMC), starches, and polymers and derivatives and mixtures thereof.

One suitable hydroxypropylmethylcellulose compound is "HPMC 2910", which is a cellulose ether having a degree of substitution of about 1.9 and a hydroxypropyl molar substitution of 0.23, and containing, based upon the total weight of the compound, from 29% to 30% methoxyl and from 7% to 12% hydroxylpropyl groups. HPMC 2910 is commercially available from the Dow Chemical Company under the tradename, "Methocel E" or "Methocel E5, " which is one grade of HPMC-2910 suitable for use in the present invention, has a viscosity of 4 to 6 cps (4 to 6 millipascal-seconds) at 20 °C in a 2% aqueous solution as determined by a Ubbelohde viscometer. Similarly, "Methocel E6 ," which is another grade of HPMC-2910 suitable for use in the present invention, has a viscosity of 5 to 7 cps ( 5 to 7 millipascal-seconds) at 20 °C in a 2% aqueous solution as determined by a Ubbelohde viscometer. "Methocel E15," which is another grade of HPMC-2910 suitable for use in the present invention, has a viscosity of about 15000 cps (15 millipascal-seconds) at 20 °C in a 2% aqueous solution as determined by a Ubbelohde viscometer. As used herein, "degree of substitution" shall mean the average number of substituent groups attached to an anhydroglucose ring, and "hydroxypropyl molar substitution" shall mean the number of moles of hydroxypropyl per mole anhydroglucose.

As used herein, "modified starches" include starches that have been modified by crosslinking, chemically modified for improved stability, or physically modified for improved solubility properties. As used herein, "pre-gelatinized starches" or "instantized starches" refers to modified starches that have been pre-wetted, then dried to enhance their cold-water solubility. Suitable modified starches are commercially available from several suppliers such as, for example, A.E. Staley Manufacturing Company, and National Starch & Chemical Company. One suitable modified starch includes the pre-gelatinized waxy maize derivative starches that are commercially available from National Starch & Chemical Company under the tradenames, "Purity Gum" and "FilmSet", and derivatives, copolymers, and mixtures thereof. Such waxy maize starches typically contain, based upon the total weight of the starch, from 0 percent to 18 percent of amylose and from 100 percent to 88 percent of amylopectin.

Suitable tapioca dextrins include those available from National Starch & Chemical Company under the tradename, "Crystal Gum" or "K-4484," and derivatives thereof such as modified food starch derived from tapioca, which is available from National Starch and Chemical under the tradename, "Purity Gum 40," and copolymers and mixtures thereof.

Examples of suitable fats include, but are not limited to hydrogenated vegetable oils such as cocoa butter, hydrogenated palm kernel oil, hydrogenated cottonseed oil, hydrogenated sunflower oil, and hydrogenated soybean oil; free fatty acids and salts thereof; and mixtures thereof.

Suitable "heat-stable, high-intensity sweeteners" shall include chemical compounds or mixtures of compounds which elicit a sweet taste at least five times sweeter than sucrose, as measured in accordance with the test method described in G.B. Patent No. 1,543,167. Typically such sweeteners are substantially free from degradants after being heated for about one hour at about 40 °C . Examples of such suitable sweeteners include, but are not limited to, sucralose, neotame, and mixtures thereof.

Sucralose, which is also known as 4,1,6'-trideoxy-galactosucrose, is a heat-stable, high-intensity sweetener that may be produced in accordance with the process disclosed in U.K. Patent No. 1,544,167, and U.S. Patent Nos. 5,136,031 and 5,498,709.

Neotame which is also known as N-(N-(3,3-dimethylbutyl)-L-a-aspartyl)-L-phenylalanine 1 methyl ester, a derivative of the dipeptide composed of the amino acids, aspartic acid and phenylalanine, is a heat-stable, high-intensity sweetener which was approved for use in the United States, July 2002 and is commercially available from The NutraSweet® Company.

Suitable non-volatile cooling agents include, but are not limited to menthyl esters, carboxamides, ureas, phosphine oxides, and mixtures thereof, to the extent that such agents are substantially free from odor or odorless vapor and thus do not lose more than about 1% by weight when placed in an open container at 50 °C for at least one hour. Typically such agents may have an average molecular weight of greater than 300 atomic molecular units (amu) or more. One example of a suitable non-volatile cooling agents is the menthyl ester mixture commercially available from International Flavors & Fragrances under the tradename, "Cooler #2".

The coating composition may also further comprise other ingredients such as, based upon the total weight of the coating solution, from 0 percent to 30 percent of a thickener; from 0 percent to 15 percent plasticizers; from 0 percent to 1 percent preservatives such as parabens; from 0 percent to 5 percent opacifying agents such as titanium dioxide; and/or from 0 percent to 15 percent colorants. See *Remington's Practice of Pharmacy,* Martin & Cook, 17^{th} ed., pp, 1625-30.

Any plasticizer known in the pharmaceutical art is suitable for use in the present invention, and may include, but not be limited to polyethylene glycol; glycerin; triethyl citrate; triethyl amine; tribuyl citrate; dibutyl sebecate; vegetable oils such as castor oil; surfactants such as polysorbates, sodium lauryl sulfates, and dioctyl-sodium sulfosuccinates; propylene glycol; monoacetate of glycerol; diacetate of glycerol; triacetate of glycerol; natural gums and mixtures thereof.

Any coloring agent suitable for use in pharmaceutical application may be used in the present invention and may include, but not be limited to azo dyes, quinopthalone dyes, triphenylmethane dyes, xanthene dyes, indigoid dyes, iron oxides, iron hydroxides, titanium dioxide, natural dyes, and mixtures thereof. More specifically, suitable colorants include, but are not limited to patent blue V, acid brilliant green BS, red 2G, azorubine, ponceau 4R, amaranth, D&C red 33, D&C red 22, D&C red 26, D&C red 28, D&C yellow 10, FD&C yellow 5, FD&C yellow 6, FD&C red 3, FD&C red 40, FD&C blue 1, FD&C blue 2, FD&C green 3, brilliant black BN, carbon black, iron oxide black, iron oxide red, iron oxide yellow, titanium dioxide, riboflavin, carotenes, antyhocyanines, turmeric, cochineal extract, clorophyllin, canthaxanthin, caramel, betanin, and mixtures thereof.

Optionally, in embodiments wherein the coating agent is a film forming polymer, a fat having a melting point less than 80 °C, a wax having a melting point less than 80° C, or a mixture thereof, the coating composition may also include, based upon the total weight of the coating composition, from 0 percent to 37 percent, e.g. from 0 percent to 25 percent of a cooling adjuvant, which includes but is not limited to cooling sugars such as sorbitol, erythritol, lactitol, maltitol, mannitol, xylitol, and mixtures thereof.

The coating composition may be applied to substrates as a coating solution in the form of a liquid or liquid with a suspended solid via dipping substrates therein or spraying substrates therewith. Such coating solutions contain a solvent in an amount, based upon the total weight of the dispersion, from 30 percent to 99 percent, for example, from 70 percent to 95 percent, or from 78 percent to 90 percent. Examples of suitable solvents include, but are not limited to water; alcohols such as methanol, ethanol, and isopropanol; organic solvents such as methylene chloride, acetone, and the like; and mixtures thereof. In one embodiment, the solvent is water. The resulting coating solution typically possesses a solids level of, based upon the total weight of the coating solution prior to removal of the solvent, from 1 percent to 70 percent, e.g. from 5 percent to 30 percent, or from 10 percent to 22 percent.

In one embodiment, the coating solution contains, based upon the total weight of the coating solution, a) from greater than 1 % and less than 70 % of a coating agent; b) from greater than 0.001 % and less than 20% a heat-stable, high-intensity sweetening agent; c) from greater than 0.001 % and less than 10 % a non-volatile cooling agent; and d) from greater than 30 % to less than 99% of a solvent.

In one embodiment, the coating composition of the present invention may be prepared by dissolving the sweetening agent, e.g., sucralose, in the solvent, e.g., water, through mixing. Optionally, a water-soluble cooling sugar may be added to mixture either at ambient conditions or at a temperature not to exceed 100 °C. The coating agent, e.g. a film-forming polymer such as hydroxypropylmethylcellulose or a mixture of film forming polymers comprising essentially of hydroxypropylmethylcellulose or polyvinyl alcohol such as that commercially available from Colorcon under the tradename, "Opa-Dry" or "Opa-Dry II" from Colorcon); the non-volatile cooling agent, e.g., menthyl ester mixture, which is commercially available from International Flavors & Fragrances under the tradename, "Cooler #2" and any other remaining ingredients may then be added thereto and mixed sufficiently to form a homogeneous mixture.

Another embodiment of the present invention is directed to a solid dosage form comprised of: a) a core; b) an optional first coating layer on the surface of the core comprised of a subcoating that substantially covers the core; and c) a second coating layer substantially covering the surface of the first coating layer, with the second coating layer comprised of the coating composition of the present invention. As used herein, "substantially covers" shall mean at least 95 percent of the surface area of the underlying substrate is covered by the given coating. For example, with respect to the first coating layer and the second coating layer, at least 95% of the surface of the first coating layer is covered by the second coating layer.

In another embodiment, the pharmaceutical dosage form is comprised of: a) a core; b) an optional first coating layer on the surface of the core comprised of a subcoating that covers a portion of the core; and c) a second coating layer that covers a portion of the surface of the first coating layer and/or the core surface, with the second coating layer comprised of the coating composition of the present invention. As used herein, "portion" shall mean a part of the dosage form having a surface area that is equal to or less than 95 percent of the surface area of the underlying substrate.

In one embodiment, the pharmaceutical dosage form contains a first portion, a second portion, and a plurality of outer coatings comprising the coating composition of the present invention, with the first portion having a first outer coating thereon and said second portion having a second outer coating thereon. In yet another embodiment, the second outer coating is visually distinct from the first outer coating by way of, for example, color, pattern texture, and/or the like.

The use of subcoatings is well known in the art and disclosed in, for example, United States Patent Nos. 3,185,626. Any composition suitable for film-coating a tablet may be used as a subcoating according to the present invention. Examples of suitable subcoatings are disclosed in United States Patent Nos. 4,683,256, 4,543,370, 4,643,894, 4,828,841, 4,725,441, 4,802,924, 5,630,871, and 6,274,162. Additional suitable subcoatings include one or more of the following ingredients: cellulose ethers such as hydroxypropylmethylcellulose, hydroxypropylcellulose, and hydroxyethylcellulose; polycarbohydrates such as xanthan gum, starch, and maltodextrin; plasticizers including for example, glycerin, polyethylene glycol, propylene glycol, dibutyl sebecate, triethyl citrate, vegetable oils such as castor oil, surfactants such as polysorbate-80, sodium lauryl sulfate and dioctyl-sodium sulfosuccinate; polycarbohydrates, pigments, and opacifiers.

In one embodiment, the subcoating may be comprised of, based upon the total weight of the subcoated tablet, from 2 percent to 8 percent, e.g. from 4 percent to 6 percent of a water-soluble cellulose ether and from 0.1 percent to 1 percent castor oil, as disclosed in detail in United States Patent No. 5,658,589. In another embodiment, the subcoating may be comprised of, based upon the total weight of the subcoating, from 20 percent to 50 percent, e.g., from 25 percent to 40 percent of IIPMC; from 45 percent to 75 percent, e.g., from 50 percent to 70 percent of maltodextrin; and from 1 percent to 10 percent, e.g., from 5 percent to 10 percent of PEG 400.

The substrates coated with the coating composition of the present invention may contain one or more active agents. The term "active agent" is used herein in a broad sense and may encompass any material that can be carried by or entrained in the system. For example, the active agent can be a pharmaceutical, nutraceutical, vitamin, dietary supplement, nutrient, herb, dyestuff, nutritional, mineral, supplement, or the like and combinations thereof.

Any number of active agents may be contained in the dosage form. The active agents may be contained in any portion of the dosage form, e.g. in the core or substrate, in the coating composition of the invention, and/or in any additional coating. In embodiments in which active agents are contained in an additional coating, the additional coating may be, for example, a first coating layer between the substrate or core and the coating of the present invention, or may be a second coating composition residing upon a portion of the core, while the coating composition of the invention resides upon a separate portion of the core. In one embodiment, one or more active agents are contained in the core of the dosage form.

The dosage forms of the present invention contain a safe and effective amount of the active agent, which means an amount of the agent that is high enough, when administered orally, to significantly positively modify the condition to be treated or prevent an adverse or unwanted condition through short-term immediate use or repeated long-term chronic use used within the scope of sound medical judgment. The safe and effective amount of the active agent will vary with the particular condition being treated; the physical condition and age of the patient being treated; the nature of concurrent therapy, if any; the duration of the treatment; the particular carrier utilized; the specific active agent(s) employed; and the like. Typically, the active agent(s) are used in an amount, based upon the total weight of the dosage form, from 0.001 percent to 99.9 percent, e.g. from 0. 1 percent to 75 percent.

The active ingredient or ingredients may be present in the dosage form in a variety of forms. For example, the active ingredient(s) may be dispersed at the molecular level, e.g. melted or dissolved, within the dosage form, or they may be in the form of particles, which in turn may be coated or uncoated. If the active ingredient is in form of particles, the particles (whether coated or uncoated) typically have an average particle size of 1 micron to 2000 microns. In one embodiment, such particles are crystals having an average particle size of about 1300 microns. In another embodiment, the particles are granules or pellets having an average particle size of 50 microns to 2000 microns, for example 50 microns to 1000 microns or from 100 microns to about 800 microns.

The active agents useful herein can be selected from classes from those in the following therapeutic categories: ace-inhibitors; alkaloids; antacids; analgesics; anabolic agents; anti-anginal drugs; anti-allergy agents; anti-arrhythmia agents; antiasthmatics; antibiotics; anticholesterolemics; anticonvulsants; anticoagulants; antidepressants; antidiarrheal preparations; anti-emetics; antihistamines; antihypertensives; anti-infectives; anti-inflammatories; antilipid agents; antimanics; anti-migraine agents; antinauseants; antipsychotics; antistroke agents; antithyroid preparations; anabolic drugs; antiobesity agents; antiparasitics; antipsychotics; antipyretics; antispasmodics; antithrombotics; antitumor agents; antitussives; antiulcer agents; anti-uricemic agents; anxiolytic agents; appetite stimulants; appetite suppressants; beta-blocking agents; bronchodilators; cardiovascular agents; cerebral dilators; chelating agents; cholecystekinin antagonists; chemotherapeutic agents; cognition activators; contraceptives; coronary dilators; cough suppressants; decongestants; deodorants; dermatological agents; diabetes agents; diuretics; emollients; enzymes; erythropoietic drugs; expectorants; fertility agents; fungicides; gastrointestinal agents; growth regulators; hormone replacement agents; hyperglycemic agents; hypoglycemic agents; ion-exchange resins; laxatives; migraine treatments; mineral supplements; mucolytics, narcotics; neuroleptics; neuromuscular drugs; non-steroidal antiinflammatory drugs (NSAIDs); nutritional additives; peripheral vasodilators; polypeptides; prostaglandins; psychotropics; renin inhibitors; respiratory stimulants; sedatives; steroids; stimulants; sympatholytics; thyroid preparations; tranquilizers; uterine relaxants; vaginal preparations; vasoconstrictors; vasodilators; vertigo agents; vitamins; wound healing agents; and others.

Active agents that may be used in the invention include, but are not limited to: acetaminophen; acetylsalicylic acid, including its buffered forms; acrivastine; albuterol and its sulfate; alkaline phosphatase; allantoin; aloe; aluminum acetate, carbonate, chlorohydrate and hydroxide; alprozolam; amino acids; aminobenzoic acid; amoxicillin; ampicillin; amsacrine; amsalog; anethole; ascorbic acid; astemizole; atenolol; azatidine and its maleate; bacitracin; balsam peru; BCNU (carmustine); beclomethasone diproprionate; benzophenones; benzquinamide and its hydrochloride; bethanechol; biotin; bisacodyl; bismuth subsalicylate; bornyl acetate; bromopheniramine and its maleate; buspirone; caffeine; calcium carbonate, casinate and hydroxide; camphor; captopril; cascara sagrada; cefaclor; cefadroxil; cephalexin; centrizine and its hydrochloride; cetirizine; cetylpyridinium chloride; chloramphenicol; chlorcyclizine hydrochloride; chlorhexidine gluconate; chloroxylenol; chloropentostatin; chlorpheniramine and its maleates and tannates; chlorpromazine; cholestyramine resin; choline bitartrate; chondrogenic stimulating protein; cimetidine; cinnamedrine hydrochloride; citalopram; clarithromycin; clemastine and its fumarate; clonidine; clorfibrate; codeine and its fumarate and phosphate; cortisone acetate; ciprofloxacin HCI; cyanocobalamin; cyclizine hydrochloride; cyproheptadine; danthron; dexbromopheniramine maleate; dextromethorphan and its hydrohalides; diazepam; dibucaine; dichloralphenazone; diclofen and its alkali metal sales; diclofenac sodium; digoxin; dihydroergotamine and its hydrogenates/mesylates; diltiazem; dimethicone; dioxybenzone; diphenhydramine and its citrate; diphenhydramine and its hydrochloride; divalproex and its alkali metal salts; docusate calcium, potassium, and sodium; doxycycline hydrate; doxylamine succinate; efaroxan; enalapril; enoxacin; ergotamine and its tartrate; erythromycin; estropipate; ethinyl estradiol; ephedrine; epinephrine bitartrate; erythropoietin; eucalyptol; famotidine; fenoprofen and its metal salts; ferrous fumarate, gluconate and sulfate; fexofenadine; fluoxetine; folic acid; fosphenytoin; 5-fluorouracil (5-FU); fluoxetine; flurbiprofen; furosemide; gabapentan; gentamicin; gemfibrozil; glipizide; glycerine; glyceryl stearate; granisetron; griseofulvin; growth hormone; guafenesin; hexylresorcinol; hydrochlorothiazide; hydrocodone and its tartrates; hydrocortisone and its acetate; 8-hydroxyquinoline sulfate; hydroxyzine and its pamoate and hydrochloride salts; ibuprofen; indomethacin; inositol; iron; isosorbide and its mono- and dinitrates; isoxicam; ketamine; kaolin; ketoprofen; lecithin; leuprolide acetate; lidocaine and its hydrochloride salt; lifinopril; liotrix; loperamide, loratadine; lovastatin; luteinizing hormore; LHRH (lutenizing hormone replacement hormone); magnesium carbonate, hydroxide, salicylate, and trisilicate; meclizine; mefenamic acid; meclofenamic acid; meclofenamate sodium; medroxyprogesterone acetate; methenamine mandelate; meperidine hydrochloride; metaproterenol sulfate; methscopolamine and its nitrates; methsergide and its maleate; methyl nicotinate; methyl salicylate; methsuximide; metoclopramide and its halides/hydrates; metronidazole; metoprotol tartrate; miconazole nitrate; minoxidil; morphine; naproxen and its alkali metal sodium salts; nifedipine; neomycin sulfate; niacin; niacinamide; nicotine; nicotinamide; nimesulide; nitroglycerine; nonoxynol-9; norethindrone and its acetate; nystatin; omega-3 polyunsaturated fatty acids; omeprazole; ondansetron and its hydrochloride; oxolinic acid; oxybenzone; oxtriphylline; padimate-O; paramethadione; pentastatin; pentaerythritol tetranitrate; pentobarbital sodium; perphenazine; phenelzine sulfate; phenindamine and its tartrate; pheniramine maleate; phenobarbital; phenolphthalein; phenylephrine and its tannates and hydrochlorides; phenylpropanolamine; phenytoin; pirmenol; piroxicam and its salts; polymicin B sulfate; potassium chloride and nitrate; prazepam; procainamide hydrochloride; procaterol; promethazine and its hydrochloride; propoxyphene and its hydrochloride and napsylate; pramiracetin; pramoxine and its hydrochloride salt; prochlorperazine and its maleate; propanolol and its hydrochloride; promethazine and its hydrochloride; propanolol; pseudoephedrine and its sulfates and hydrochlorides; pyridoxine; pyrolamine and its hydrochlorides and tannates; quinapril; quinidine gluconate and sulfate; quinestrol; ralitoline; ranitadine; resorcinol; riboflavin; salicylic acid; scopolamine; sesame oil; sodium bicarbonate, citrate, and fluoride; sodium and sulfate; quinestrol; ralitoline; ranitadine; resorcinol; riboflavin; salicylic acid; scopolamine; sesame oil; sodium bicarbonate, citrate, and fluoride; sodium monofluorophosphate; sucralfate; sulfanethoxazole; sulfasalazine; sulfur; sumatriptan and its succinate; tacrine and its hydrochloride; theophylline; terfenadine; thiethylperazine and its maleate; timolol and its maleate; thioperidone; tramadol; trimetrexate; triazolam; tretinoin; tetracycline hydrochloride; tolmetin; tolnaftate; trimethobenzamide and its hydrochloride; tripelennamine and its hydrochloride; tripolidine hydrochloride; undccylenic acid; vancomycin; verapamil HCl, vidaribine phosphate; vitamins A, B₁₋₁₂, C, D, E, and K; xylometazoline hydrochloride; zinc. Active agents may further include, but are not limited to food or herbal extracts; insoluble metal and mineral hydroxides, carbonates, oxides, polycarbophils, and salts thereof; adsorbates of active drugs on a magnesium trisilicate base and on a magnesium aluminum silicate base, and mixtures thereof.

Any of the active agents set forth above, pharmaceutically acceptable salts thereof, pharmaceutically acceptable enantiomers thereof, and mixtures thereof are also suitable for use in the present invention.

In one embodiment, the dosage form contains an active agent suitable for use in the treatment of symptoms of cough, cold, cold-like, allergy, and/or flu in a mammal. In another embodiment, the dosage form may contain guaifenesin in combination with acetaminophen and pseudoephedrine HCl. In another alternative embodiment, the dosage form may be comprised of a placebo core (containing, for example, lactose and cellulose), which does not contain an active agent.

The average weight gain of dried dosage form after application of the coating composition of the present invention thereto is, based upon the total weight of the dried coated dosage form, from 0.25 percent to 10 percent, e.g. from 2 percent to 4 percent. The average thickness of the dried layer of the coating composition typically is from 30 microns to 400 microns. However, one skilled in the art would readily appreciate without undue experimentation that the thickness of the coating composition may be varied in order to provide a smoother, easier to swallow, dosage form; to change the coating aesthetics; or to achieve a desired dissolution profile.

The coating composition of the present invention may be applied to the core or substrates via any methods known in the art such as, for example, spray coating, pan coating, dip coating, and molding as disclosed in, for example, McGinity, "Aqueous Polymeric Coatings for Pharmaceutical Dosage Forms" from the series "Drugs and the Pharmaceutical Sciences" (Volume 36, 1989).

In one embodiment, the coating solution may be applied to a caplet or tablet core via spraying in a pan coater using heat at a temperature sufficient to remove any solvent that may be in the coating solution. Alternatively, part or all of the core may be coated with the coating solution by dipping the substrate therein. In a further alternative embodiment, the coating solution may be sprayed onto a particle or plurality of particles, which then could be incorporated into a larger solid dosage form, e.g. compressed into a chewable tablet.

We have unexpectedly found that the coating composition of the dosage form of the present invention effectively tastemasks any unpleasant taste that might otherwise have been associated with uncoated or conventional film-coated dosage forms. The user of a dosage form coated with the coating composition also experiences a mild cooling sensation in the throat and/or mouth without any associated, unpleasant aroma/odor or polarizing taste as may be experienced by use of coatings containing menthol and other intense mint-like volatile flavors. Surprisingly, the cooling sensation, which primarily occurs after swallowing, can be "reactivated" or "re-intensified" through choice by the user for several minutes after consumption by simply taking a slightly deeper or slightly exaggerated breath despite the absence of the solid dosage form in the mouth or throat. The coating composition also enhanced the appeal of a particular medicine over conventional film-coated medicines so that users do not avoid taking their medicine.

A further advantage of the coating compositions of the dosage form of the present invention is that the resulting coated dosage form has a sweet taste without the inclusion of sugar. Not only will this improve a patient's compliance with taking the prescribed pharmaceutical, but also it will not promote tooth decay or increase caloric intake like sugar coated products. Moreover, the sugar-free coating is especially suitable for diabetic users and those restricting sugars from their diets. In addition, sugar coatings disadvantageously are relatively less stable than sucralose coatings, and thus often react with other components in the coating and discolor. Yet further, the sucralose coatings of the present invention do not provide a nutritional source for potential microbial contamination as do sugar coated products.

Yet a further advantage is that because the coating composition of the dosage form of the present invention is substantially free of volatile cooling agents such as mints, it provides the user with a cooling benefit without aggravating conditions such as gastroesphageal reflux disease commonly referred to as "GERD".

And yet another advantage was that the inclusion of the non-volatile cooling agent in the coating composition decreased its pre-coating deaeration time to less than one-third of the deaeration time required by other film forming polymeric coating formulations. "Deaeration, " as used herein, shall mean the substantial absence of visually discernable foam.

Several examples are set forth below to further illustrate the nature of the invention and the manner of carrying it out.

### EXAMPLES

### Example 1. Preparation of a Cool Coating Solution

A cool coating solution having the components set forth below was prepared as follows:

### Solution ingredients:

15.32 mg Opadry II Green (85F11782) *
   * Blend containing Polyvinyl Alcohol, Titanium Dioxide, Polyethylene Glycol, Talc, FD&C Blue#1 Aluminum Lake, D&C Yellow #10 Aluminum Lake, FD&C Red #40 Aluminum Lake, which is commercially available from Colorcon
5.39 mg Mannitol, NF
0.500 mg Sucralose , NF***
   *** Sucralose was obtained from McNeil Nutritional Products Division of McNEIL-PPC, Inc.
2.31 mg menthyl ester blend
   **Blend commercially available from IFF, Inc. under the tradename. "Cooler #2 Liquid SN 069450. "

### Solution Preparation:

All of the ingredients were added to 94 mg (per tablet basis) water with continuous mixing for 10 minutes until homogenous. The resulting homogenous mixture contained only a small amount of visibly discernable surface foam on the surface. After 10 minutes, the foam was dissipated, and the resulting solution was deemed ready for spraying.

### Example 2: Preparation of Uncoated, solid Core

555 mg of Active Agents comprised of:
   325 mg acetaminophen
   200 mg guaifenesin
   30 mg pseudoephedrine HCl
235 mg of In-actives comprised of
   6.2 mg polyvinylpyrrolidone
   6.5 mg sodium starch glycolate
   119.3 mg cellulose
   91.0 mg starch
   1.9 mg silicon dioxide
   8.0 mg stearic acid
   2.1 mg magnesium stearate

Each of the active agents were wet-granulated and dried independently as follows:

### a) Guaifenesin:

200 mg of guaifenensin were wet-granulated using 6.2 mg polyvinylpyrrolidone in a high-shear granulator, then transferred and dried in a fluid bed granulator.

### b) Acetaminophen:

325 mg of acetaminophen were wet-granulated then dried using 4.2 mg sodium starch glycolate, 32.5 mg of starch, and 26 mg of cellulose in a fluid bed granulator at a temperature of about 75 °C to about 85 °C.

### c) Pseudoephedrine:

30 mg of pseudoephedrine were wet-granulated using 2.3 mg sodium starch glycolate, 15.7 mg of cellulose, 58.5 mg of starch in a fluid bed granulator.

The three granulations were then blended with 77.6 mg of cellulose, 2.1 mg of magnesium stearate, 1.9 mg silicon dioxide, and 8.0 mg of stearic acid, then the resulting mixtures was compressed into a solid having a caplet shape on a Fette 1200 rotary tablet machine.

### Example 3: Preparation of "Plain" Film Coating Solution

A coating solution having the components set forth below was prepared as follows:

### Solution ingredients:

22.7 mg Opadry II Green (85F11782) *
   * Blend containing Polyvinyl Alcohol, Titanium Dioxide, Polyethylene Glycol, Talc, FD&C Blue#1 Aluminum Lake, D&C Yellow #10 Aluminum Lake, FD&C Red #40 Aluminum Lake, which is commercially available from Colorcon,

### Solution Preparation:

The OpaDry II Green blend was added to 90.8 mg (per tablet basis) water with continuous mixing in a 1.5 liter vessel using a Janke & Kunkle RW 20 DZM mixer for about 30 minutes. At that time the solution was homogeneous and substantially free from visually discernable foam.

### Example 4. Preparation of Uncoated Placebo Cores

### Core Ingredients (inactive):

480 mg lactose monohydrate
292.5 mg cellulose
7.5 mg magnesium stearate

The above inactive ingredients were blended dry using a 1 cubic foot V-blender for 5 minutes at a speed of about 25 rpm. The mixture was then compressed via the procedure of Example 2 to form caplets having the same size as those in Example 2.

### Example 5. Spray Coating Substrates with Coating Solutions

Example 5 A. (Cool-Coated Active) Approximately 10000 (or about half) of cores produced in accordance with Example 2 were spray coated with the coating solution of Example 1 via the use of a 24 inch Accela-Cota® Pan Coater. The solution was sprayed at a rate of 30-60 g/min on to each core, then each coated core was dried using heated air sufficient in order to achieve a targeted bed temperature of approximately 40°C to 50°C during both the spraying and drying phases.

Example 5B: (Cool-Coated Placebo) Approximately 9000 (or about half) of cores produced in accordance with Example 4 were spray coated with the coating solution of Example 1 via the use of a 24 inch Accela-Cota® Pan Coater . The solution was sprayed at a rate of 30-60 g/min on to each core, then each coated core was dried using heated air sufficient in order to achieve a targeted bed temperature of approximately 40°C to 50°C during both the spraying and drying phases.

Example 5C: (Plain Coated Active) Approximately 9000 (or about half) of cores produced in accordance with Example 2 were spray coated with the coating solution of Example 3 via the use of a 24 inch Accela-Cota® Pan Coater . The solution was sprayed at a rate of 30-60 g/min on to each core, then each coated core was dried using heated air sufficient in order to achieve a targeted bed temperature of approximately 40°C to 50°C during both the spraying and drying phases.

Example 5D: (Plain Coated Placebo) Approximately 16000 cores produced in accordance with Example 4 were spray coated with the coating solution of Example 3 via the use of a 24 inch Accela-Cota® Pan Coater . The solution was sprayed at a rate of 30-60 g/min on to each core, then each coated core was dried using heated air sufficient in order to achieve a targeted bed temperature of approximately 40°C to 50°C during both the spraying and drying phases.

### Example 6: Comparison of Coated and Uncoated Substrates

Three studies were performed using Examples 5A, 5B, 5C and 2 (as the uncoated substrate).

### Example 6A: Comparison of "Plain" Film Coated Placebo with "Cool" Coated Placebo

In a blinded study, 2 identically colored coated caplets prepared in accordance with Example 5B ("cool" coated placebo) were independently administered to two hundred and seventy (270) adults. After each sample, each adult evaluated the caplets for the attributes listed in Table A.

This procedure was repeated with 2 identically-colored, coated caplets prepared in accordance with Example 5D ("plain" coated placebo). The results are set forth in Table A below:

**Table A: Evaluation of "Plain" Coated Placebo with "Cool" Coated Placebo**

| **Attribute Evaluated** | **"Plain" Coated Placebo** | **Cool Coated Placebo** |
|---|---|---|
| Overall Preference (Number of individuals out of 270) | 85 | 185 |
| Overall Liking* | 4.3 (1.3) | 4.8 (1.4)** |
| Taste* | 4.0 (1.0) | 4.8 (1.3)** |
| Aftertaste* | 4.0 (0.8) | 4.6 (1.1) ** |
| Size* | 3.7 (0.6) | 3.6 (0.6) |
| Sweetness Level* | 1.4 (0.8) | 2.5 (1.0)** |
| Cooling Level* | 1.4 (0.8) | 1.9 (1.0) ** |
| Ease of Swallow* | 2.6 (1.1) | 2.6 (1.0) |

| | | |
|---|---|---|
| *Scales for property evaluation: overall liking: (1- dislike very much) <-----→(7 - like very much); taste: (1 - dislike very much) <-----→(7 - like very much); size: (1 - much too small) <-----→(5 - much too large); sweetness: level: (1- none) <-----→(5 - high); cooling level: (1 - none) <-----→(5 - high); ease of swallow: (1- very easy) <-----→(5 - very difficult); aftertaste: (1- very unpleasant) <-----→(7 - very pleasant); ** Statistically significant difference @ p ≤0.001. | | |

This study showed that the caplet coated with the cool coating composition of the present invention was preferred by 69% of the adults over the "plain" (or "conventional") film coated caplet. The caplet coated with the cool coating composition also received statistically more favorable ratings for Overall Liking, Taste, Size, Sweetness Level, and Aftertaste, and was comparable to film-coated caplets with respect to Ease of Swallow and Size.

### Example 6B: Comparison of "Plain " Film Coated and "Cool" Coated Substrates Containing Active Agents

In a blinded study, 2 coated caplets prepared in accordance with Example 5A (cool coated, active core) and 2 coated caplets prepared in accordance with Example 5C (plain coated, active core) were independently administered to one hundred and seventeen (117) adults as follows: Each adult was instructed to put one of two identically coated caplets prepared in accordance with Example 5C from an envelope into their mouth, to consume water as if they were going to swallow the caplet, then to expectorate the caplet. This method was repeated with the other coated caplet in the envelope. After each adult evaluated these caplets for the attributes listed in Table C, each adult then evaluated two identically coated caplets prepared in accordance with Example 5A in a similar fashion. The results are set forth in Table B below:

**Table B: Evaluation of "Plain" Film coated verses Coolant coated caplets**

| **Attribute Evaluated** | **"Plain"-Coated, Active Substrate (caplet)** | **"Cool"-Coated, Active Substrate (caplet)** |
|---|---|---|
| Overall Preference (Number of individuals out of 117) | 8 | 109 |
| Overall Liking* | 2.5 (1.4) | 5.1 (1.5)** |
| Taste* | 2.3 (1.3) | 5.2 (1.5)** |
| Aftertaste* | 2.4 (1.3) | 4.8 (1.5)** |
| Sweetness Level* | 1.1 (0.4) | 2.9 (0.9)** |
| Cooling Level* | 1.4 (0.7) | 2.5 (1.0)** |

| | | |
|---|---|---|
| *Scales for property evaluation: 1) overall liking: (1- dislike very much) <--------------- →(7 - like very much); 2 taste: (1 - dislike very much) <--------------- →(7 - like very much); 3) aftertaste: (1 - very unpleasant) <--------------- →(7 - very pleasant); 4) sweetness: level: (1- none) <--------------- →(5 - high); 5) cooling level: (1- none) <--------------- →(5 - high); **Statistically significant difference @ p ≤0.001. | | |

This study showed that 93% of the adults preferred the active-containing caplets coated with the coating solution of the present invention over the active-containing caplets coated with the "plain" film coating. The active-containing caplets coated with the coating solution also received statistically more favorable ratings for every attribute evaluated: Overall Liking, Taste, Size, Sweetness Level, and Aftertaste.

### Example 6C: Comparison of Uncoated, Active-containing Substrate with Cool Coated Substrate

In a blinded study, 2 uncoated, active-containing caplets prepared in accordance with Example 2 were independently administered to one hundred and thirty three (133) adults as follows: Each adult was instructed to put one of two identical, uncoated, active-containing caplets from an envelope into their mouth, to consume water as if they were going to swallow the caplet, then to expectorate the caplet. This method was repeated with the other uncoated, active-containing caplet in the envelope. After each adult evaluated these caplets for the attributes listed in Table C, two "cool" coated, active-containing caplets prepared in accordance with Example 5A were independently administered to each adult, respectively, in a similar fashion. Each adult then similarly evaluated these latter two caplets. The results are set forth in Table C below:

**Table C: Evaluation of Uncoated, Active-Containing caplet with "Cool"coated, Active-containing caplet**

| **Attribute Evaluated** | **Uncoated Substrate (caplet)** | **"Cool" Coated Substrate (caplet)** |
|---|---|---|
| Overall Preference (Number of individuals out of 133) | 14 | 119 |
| Overall Liking* | 2.2 (1.3) | 5.1 (1.5)** |
| Taste* | 2.1 (1.2) | 5.2 (1.5)** |
| Aftertaste* | 2.1 (1.2) | 4.8 (1.5)** |
| Sweetness Level* | 1.1 (0.5) | 2.9 (0.9)** |
| Cooling Level* | 1.3 (0.7) | 2.5 (1.0)** |

| | | |
|---|---|---|
| *Scales for property evaluation: 1) overall liking: (1 - dislike very much) <------------------→(7 - like very much); 2 taste: (1-dislike very much) <------------------→(7 - like very much); 3) aftertaste: (1 - very unpleasant) <------------------→(7 - very pleasant); 4) sweetness: level: (1 - none) <------------------→(5 - high); 5) cooling level: (1- none) <------------------→(5 - high); ** Statistically significant difference @ p ≤0.001. | | |

This study showed that 89% of the adults preferred the active-containing caplet coated with the cool coating composition of the present invention over the uncoated caplet. The active-containing caplet coated with the cool coating composition also received statistically more favorable ratings for every attribute evaluated: Overall Liking, Taste, Size, Sweetness Level, and Aftertaste.

## Claims

1. A pharmaceutical solid dosage form comprising a core and an outer coating, said outer coating comprising the coating composition comprising:
a) a coating agent;
b) a heat-stable, high-intensity sweetening agent; and
c) a non-volatile cooling agent,
wherein the coating agent is a crystallizable carbohydrate; a film forming polymer; a fat having a melting point less than 80°C;a wax having a melting point less than 80°C; or a mixture thereof.

2. The dosage form of claim 1, wherein the crystallizable carbohydrate is sucrose.

3. The dosage form of claim 1 or 2, wherein the coating agent is:
a.) a film forming polymer which is polyvinylalcohol (PVA), hydroxypropyl starch, hydroxyethyl starch, pullulan, methylethyl starch, carboxymethyl starch, methylcellulose, hydroxypropylcellulose (HPC), hydroxyethylmethylcellulose (HEMC), hydroxypropylmethylcellulose (HPMC), hydroxybutylmethylcellulose (HBMC), hydroxyethylethylcellulose (HEEC), hydroxyethylhydroxypropylmethyl cellulose (HEMPMC), cellulose acetate (CA), cellulose acetate phthalate (CAP), carboxymethylcellulose (CMC), a starch or a polymer, derivative or mixture thereof; or
b.) a wax having a melting point less than 80°C; or
c.) a mixture thereof.

4. The dosage form of any one of claims 1 to 3, wherein the sweetening agent is sucralose.

5. The dosage form of any one of claims 1 to 4, wherein the non-volatile cooling agent is comprised of at least one menthyl ester having an average molecular weight of greater than about 300 atomic molecular units.

6. The dosage form of any one of claims 1 to 5 further comprising, based upon the total dry weight of the coating composition from greater than 0 % and less than 25 % of a cooling adjuvant.

7. The dosage form of claim 6, wherein the cooling adjuvant is a cooling sugar, which preferably is sorbitol, erythritol, lactitol, maltitol, mannitol, xylitol or a mixture thereof.

8. A dosage form of any one of claims 1 to 7 comprising a core, a subcoating substantially covering said core, and an outer coating substantially covering said subcoating.

## Patentansprüche

1. Pharmazeutische feste Darreichungsform, umfassend einen Kern und einen äußeren Überzug, wobei der äußere Überzug die Überzugszusammensetzung umfaßt, umfassend:
a) ein Überzugsmittel;
b) ein hitzestabiles, hochintensives Süßungsmittel; und
c) ein nicht-flüchtiges Kühlmittel,
wobei das Überzugsmittel ein kristallisierbares Kohlenhydrat; ein filmbildendes Polymer; ein Fett, das einen Schmelzpunkt von weniger als 80°C aufweist; ein Wachs, das einen Schmelzpunkt von weniger als 80°C aufweist, oder eine Mischung davon ist.

2. Darreichungsform nach Anspruch 1, wobei das kristallisierbare Kohlenhydrat Saccharose ist.

3. Darreichungsform nach Anspruch 1 oder 2, wobei das Überzugsmittel:
a) ein filmbildendes Polymer, das Polyvinylalkohol (PVA), Hydroxypropylstärke, Hydroxyethylstärke, Pullulan, Methylethylstärke, Carboxymethylstärke, Methylcellulose, Hydroxypropylcellulose (HPC), Hydroxyethylmethylcellulose (HEMC), Hydroxypropylmethylcellulose (HPMC), Hydroxybutylmethylcellulose (HBMC), Hydroxyethylethylcellulose (HEEC), Hydroxyethylhydroxypropylmethylcellulose (HEMPMC), Celluloseacetat (CA), Celluloseacetatphthalat (CAP), Carboxymethylcellulose (CMC), eine Stärke oder ein Polymer, Derivat oder Mischung davon ist; oder
b) ein Wachs, das einen Schmelzpunkt von weniger als 80°C aufweist; oder
c) eine Mischung davon ist.

4. Darreichungsform nach einem der Ansprüche 1 bis 3, wobei das Süßungsmittel Sucralose ist.

5. Darreichungsform nach einem der Ansprüche 1 bis 4, wobei das nicht-flüchtige Kühlmittel mindestens einen Methylester umfaßt, der ein durchschnittliches Molekulargewicht von mehr als ungefähr 300 atomaren Masseneinheiten aufweist.

6. Darreichungsform nach einem der Ansprüche 1 bis 5, weiter umfassend mehr als 0% und weniger als 25% eines Kühlhilfsmittels, bezogen auf das Gesamttrockengewicht der Überzugszusammensetzung.

7. Darreichungsform nach Anspruch 6, wobei das Kühlhilfsmittel ein Kältezucker ist, der vorzugsweise Sorbitol, Erythritol, Lactitol, Maltitol, Mannitol, Xylitol oder eine Mischung davon ist.

8. Darreichungsform nach einem der Ansprüche 1 bis 7, umfassend einen Kern, einen Zwischenüberzug, der im wesentlichen den Kern ummantelt, und einen äußeren Überzug, der im wesentlichen den Zwischenüberzug ummantelt.

## Revendications

1. Forme posologique pharmaceutique solide comprenant un noyau et un revêtement extérieur, ledit revêtement extérieur comprenant la composition de revêtement comprenant:
a) un agent de revêtement ;
b) un agent édulcorant thermostable de haute intensité; et
c) un agent de refroidissement non volatile ;
dans laquelle l'agent de revêtement est un hydrate de carbone cristallisable; un polymère filmogène ; une graisse ayant un point de fusion inférieur à 80°C ; une cire ayant un point de fusion inférieur à 80°C ; ou un mélange de ceux-ci.

2. Forme posologique selon la revendication 1,
dans laquelle l'hydrate de carbone cristallisable est le saccharose.

3. Forme posologique selon la revendication 1 ou la revendication 2, dans laquelle l'agent de revêtement est :
a) un polymère filmogène qui est l'alcool polyvinylique (PVA), un amidon hydroxypropylique, un amidon hydroxyéthylique, le pullulane, un amidon méthyléthylique, un amidon carboxyméthylique, la méthylcellulose, l'hydroxypropylcellulose (HPC), l'hydroxyéthylméthylcellulose (HEMC), l'hydroxypropylméthylcellulose (HPMC), l'hydroxybutylméthylcellulose (HBMC), l'hydroxyéthyléthylcellulose (HEEC), l'hydroxyéthylhydroxypropylméthylcellulose (HEMPMC), l'acétate de cellulose (CA), l'acétatephtalate de cellulose (CAP), la carboxyméthylcellulose (CMC), un amidon ou un polymère, un dérivé ou un mélange de ceux-ci ; ou
b) une cire ayant un point de fusion inférieur à 80°C ; ou
c) un mélange de ceux-ci.

4. Forme posologique selon l'une quelconque des revendications 1 à 3, dans laquelle l'agent édulcorant est le sucralose.

5. Forme posologique selon l'une quelconque des revendications 1 à 4, dans laquelle l'agent de refroidissement non volatile est composé d'au moins un ester menthyle ayant un poids moléculaire moyen supérieur à environ 300 unités moléculaires atomiques.

6. Forme posologique selon l'une quelconque des revendications 1 à 5 comprenant en outre, sur la base du poids sec total de la composition de revêtement d'une valeur supérieure à 0% à une valeur inférieure à 25 % d'un adjuvant de refroidissement.

7. Forme posologique selon la revendication 6, dans laquelle l'adjuvant de refroidissement est un sucre de refroidissement, qui de préférence est le sorbitol, l'érythritol, le lactitol, le maltitol, le mannitol, le xylitol ou un mélange de ceux-ci.

8. Forme posologique selon l'une quelconque des revendications 1 à 7 comprenant un noyau, un sous-revêtement couvrant essentiellement ledit noyau, et un revêtement extérieur couvrant essentiellement ledit sous-revêtement.
